(19) Europäisches Patentamt
European Patent Office
Office européen des brevets

(11) EP 1 932 507 A2

(12) EUROPEAN PATENT APPLICATION

(43) Date of publication:
**18.06.2008 Bulletin 2008/25**

(21) Application number: **07023842.3**

(22) Date of filing: **10.12.2007**

(51) Int Cl.:
***A61K 8/34*** *(2006.01)* ***A61K 8/89*** *(2006.01)*
***A61Q 5/00*** *(2006.01)* ***A61Q 5/06*** *(2006.01)*

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **12.12.2006 JP 2006334791**

(71) Applicant: **KAO CORPORATION**
**Tokyo 103-8210 (JP)**

(72) Inventor: **Nishizawa, Eiichi**
**Tokyo 131-8501 (JP)**

(74) Representative: **HOFFMANN EITLE**
**Patent- und Rechtsanwälte**
**Arabellastrasse 4**
**81925 München (DE)**

# (54) Hair treatment method

(57) A hair treatment method in which a hair treatment agent including (A) water, (B) a hydrophobic silicone, (C) 0.5% by weight or more of a lower alcohol and (D) a surfactant is applied to the hair, and immediately thereafter the hair is bundled and secured with a holding device.



Printed by Jouve, 75001 PARIS (FR)

## Description

### FIELD OF THE INVENTION

The present invention relates to a hair treatment method for forming neatly gathered hair.

### BACKGROUND OF THE INVENTION

Gathered hair is a generic designation for a hairstyle in which the hair is bundled and secured with a holding device. Gathered hair can provide a bright, cheerful and clean image, and hence always occupies a high standing in female hairstyles. Gathered hair includes variations depending on the handling of the bundled hair such as a ponytail in which the hair is bundled at the back of the head and allowed to hang freely from that point, and a chignon in which the hair is bundled and rolled into a bun at the top of the head. It is to be noted that a common fundamental factor for forming neatly gathered hair is that the hair is neatly bundled and secured with a holding device.

In forming gathered hair, there is generally adopted a hair treatment method in which dried hair is bundled and secured with a holding device. However, in dried hair, hair near the roots stand up from the hair roots along the growing direction, and consequently, it is difficult to run a comb or fingers through the hair so as to make the hair lie down flat and parallel to the scalp, and to align the hair in one direction. Additionally, in dried hair, among the hairs in the area between the hair roots and the portion to be bundled with a holding device, some short hairs (loose hairs) tend to escape from the others or some short hairs (flyaway hairs) tend to go in various directions, and hence the hair cannot be neatly bundled to be secured with a holding device.

In some cases, in order to facilitate the hair bundling, there is adopted a hair treatment method in which the hair is moistened with water, and immediately thereafter the hair is bundled and secured with a holding device. According to this method, the moistening with water breaks the hydrogen bonds between the protein fibers inside the hairs to soften the hairs themselves, and hence the hair near the roots can be readily made to lie down flat and parallel to the scalp, and the hair streaks can be neatly aligned in one direction. Additionally, because water is present between the hairs, the capillary action of the water generates the attractive force exerting between the hairs (capillary attractive force), and hence the loose hairs or the flyaway hairs occur less among the hairs in the area between the roots and the portion of the hair bundled with a holding device. However, the thorough moistening of all the hairs in order to suppress the loose hairs and the flyaway hairs elongates the time required for drying the hair after having been bundled, and hence causes some inconveniences such that it is not possible to go outdoors immediately after the hair bundling. Recently, a longer hairstyle tends to be increasingly popular, and this tendency has become more remarkable. Additionally, the retention of the gathered hair style after drying has not been sufficiently satisfactory.

Additionally, the recent expanding use of hair color treatment and permanent wave treatment causes severe damage to the hair, and hence gives rise to a tendency such that the surface of the hair is hydrophilized. Accordingly, when the hair is moistened with water, an excessively strong capillary force is exerted between the hairs, and hence the hair tend to make a rigid and stiff bundle. Consequently, there occurs the following problem: even when the hair streaks are attempted to be aligned by combing after the hair has been moistened with water in order to facilitate the bundling of the hair, it is difficult to run a comb or fingers smoothly through the hair, it is thereby difficult to align the hair streaks, and hence the hair cannot be neatly bundled and secured.

Additionally, recently, a hair cut technique to thin out the hair in order to reduce the volume of the hair has come to be widely used, and hence long hairs and short hairs tend to be present in a mixed manner. This also causes a problem such that even when the hair is moistened with water, loose hairs and flyaway hairs tend to be caused, and the hair cannot be neatly bundled and secured. Additionally, such hair also involves a problem such that when only the surface of the hair portion desired to be bundled is locally moistened, loose hairs and flyaway hairs can hardly be suppressed.

Under these circumstances, there has been demanded a hair treatment method in which: even when the hair is moistened in order to impart flexibility and capillary attractive force to the hair, the hair does not become stiff, and fingers or a comb can be smoothly run through the hair, and hence the hair streaks can be aligned while loose hairs and flyaway hairs are being suppressed without failure; neatly gathered hair can be easily formed by bundling the hair immediately after the moistening and thereafter securing the bundled hair with a holding device; the moistened hair can be quickly and naturally dried; and after drying, the gathered hairstyle is retained.

### SUMMARY OF THE INVENTION

The present invention provides a hair treatment method in which a hair treatment agent including (A) water, (B) a hydrophobic silicone, (C) 0.5% by weight or more of a lower alcohol and (D) a surfactant is applied to the hair, and immediately thereafter the hair is bundled and secured with a holding device.

DETAILED DESCRIPTION OF THE INVENTION

The present invention relates to a hair treatment method capable of forming neatly gathered hair without involving the occurrence of loose hairs and flyaway hairs.

According to the hair treatment method of the present invention, even when the hair is wetted with a hair treatment agent in order to impart flexibility and capillary attractive force to the hair, the hair does not become stiff, and hence fingers or a comb can be run smoothly through the hair, and hair streaks can be aligned while loose hairs and flyaway hairs are being suppressed without failure. Consequently, the hair is immediately bundled and thereafter secured with a holding device, and thus neatly gathered hair can be easily formed. Additionally, the wet hair can be dried quickly and naturally, and hence immediately after forming gathered hair, one may go outdoors. Furthermore, after drying, the gathered hair style is retained satisfactorily.

[Hair Treatment Method]

When gathered hair is formed by using a hair treatment agent including water, a hydrophobic silicone, a lower alcohol and a surfactant, the hair treatment agent is applied to the hair, and thereafter the hair streaks are aligned with fingers, a comb or the like, and immediately thereafter, the hair has only to be bundled and secured with a holding device.

From the viewpoint that the wetted hair is dried quickly and naturally after hair bundling, and the hair streaks can be aligned while loose hairs and flyaway hairs are being suppressed without failure, the way of applying the hair treatment agent is such that the hair treatment agent is not applied to the whole hair or the tip of the hairs, but is preferably applied to the area from the roots of the hairs to the portion to be bundled with a holding device, and more preferably applied as a mist or otherwise applied thinly. It is preferable to make the hair treatment agent applicable not only to the surface of the hair, but also relatively wholly and evenly to the hairs in such a way that the hair treatment agent can be evenly applied to the inner side of the hair and to the hairs in the area between the roots and the portion to be bundled. A specific application method for that purpose is preferably such that the hair is once lifted up and then the hair treatment agent is applied to the hair, wherein the hair portion to be applied with the hair treatment agent is preferably made to fall within the area from the roots to the portion to be secured with a holding device.

From the viewpoint of a thin application, preferable forms of the hair treatment agent used in the present invention include a pump spray, an aerosol spray, a pump foam, an aerosol foam, a gel and a lotion. From the viewpoint of an easy, thin and even application, more preferable are a pump spray, an aerosol spray, a pump foam and an aerosol foam. Moreover, from the viewpoint of application as a mist, preferable are a pump spray and an aerosol spray, and more preferable is a pump spray.

In the present invention, the "thin application" means that a hair treatment agent is applied to the hair in such a area that the bath ratio of the hair treatment agent: the hair = 0.01:1 to 3:1, preferably 0.04:1 to 2:1, more preferably 0.08:1 to 1:1. When the hair treatment agent is applied in such a bath ratio range, the agent does not fall in drops, a comb can be run smoothly through the hair, the occurrence of loose hairs and flyaway hairs can be suppressed, and moreover, an advantage is provided that the hair is dried immediately after hair bundling.

In the present invention, the term "directly" means that after application of a hair treatment agent, the hair is secured immediately without applying a hair dryer while the hair is bundled and secured with a holding device. The term "immediately" refers to any time point falling within a time period between after the application of the hair treatment agent and before the completion of natural drying; specifically, as measured after the final operation of applying the hair treatment agent, the time point falls within one hour, preferably within 30 minutes, more preferably within 15 minutes, even more preferably within 10 minutes, and even more preferably within 5 minutes. Examples of the holding device used for securing include a rubber band, a rubber string, a hairpin, an ornamental hairpin, a barrette and a ribbon; any device capable of securing may be adopted.

At the time of securing, the streaks of the hair applied with the hair treatment agent can be aligned with fingers or a comb. The way of running fingers or a comb through the hair is preferably such that the hair streaks are aligned while the tips of the fingers or the tooth ends of a comb are being in contact with the scalp but not being kept away from the scalp, from the viewpoint that the hair streaks are aligned while the occurrence of loose hairs and flyaway hairs is being suppressed in such a way that the hairs are made to lie down, from the roots thereof, flat and parallel to the scalp.

Additionally, the retention of gathered hair can also be further improved by setting the hairstyle by treating the hair with a second hair treatment agent to be described below after the gathered hair has been formed as described above by treating the hair with a first hair treatment agent and securing the hair with a holding device.

[Hair Treatment Agent]

The hair treatment agent used in the present invention contains water as component (A) in order to impart the hair flexibility and attractive force between hairs. The content of water is 10 to 99% by weight, more preferably 30 to

95% by weight and even more preferably 60 to 90% by weight, from the viewpoint that when the hair streaks are aligned with fingers or a comb, loose hairs and flyaway hairs are suppressed by wetting the hair.

From the viewpoint of a thin and even application, the form of the hair treatment agent used in the present invention may be appropriately selected from the forms such as a liquid, gel, paste, cream and wax. The viscosity of the hair treatment agent used in the present invention is preferably 100 mPa·s or less, more preferably 50 mPa·s or less and even more preferably 10 mPa·s or less. It is to be noted that the measurement of the viscosity is to be carried out with a BM viscometer manufactured by Tokyo Keiki Co., Ltd.

Examples of the second hair treatment agent include common hair styling agents such as a wax, foam, gel and spray (among them, spray is preferred). Preferable as a wax are the hard types mixed with solid fat. Examples of the solid fat include paraffin, microcrystalline wax and vaseline. Preferable as a spray, gel and foam are the hard types mixed with set polymers.

The hair treatment agent used in the present invention contains component (B), namely, a hydrophobic silicone. The hydrophobic silicone as component (B) provides an advantageous effect that, at the time of aligning the hair streaks, hair is made smooth by decreasing the hair surface friction resistance of the wetted hair to suppress the hair entanglement and to thereby facilitate bundling, and another advantageous effect is that a soft set coating is formed on the surface of the dried hair after finishing, and consequently the occurrence of loose hairs and hair bulging with time is suppressed to improve the retention of the hairstyle. In the present specification, among silicones, the hydrophobic silicones mean silicones other than the silicones which become transparent and uniform as observed by visual inspection after being subjected to the following operations.

A hydrophobic silicone as referred to herein means a silicone which does not provide a transparent mixture (segregation, gelation, or white turbidity) after being mixed with an evaluation liquid (purified water, preferably a 50% by weight aqueous solution of ethanol, most preferably a 95% by weight aqueous solution of ethanol) in a weight ratio of 1:1 at room temperature, and allowed to stand for 5 minutes at room temperature. Among the commercially available silicones, the hydrophobic silicones as referred to herein mean emulsion type silicones and silicones mixed with organic solvents to be hydrophobic.

Examples of the hydrophobic silicones include dimethylpolysiloxane, polyether-modified silicones (HLB: less than 11), amino-modified silicones, carboxy-modified silicones, methylphenylpolysiloxane, fatty acid-modified silicones, alcohol-modified silicones, aliphatic alcohol-modified silicones, epoxy-modified silicones, fluorine-modified silicones, cyclic silicones and alkyl-modified silicones. Among them, dimethylpolysiloxanes, polyether-modified silicones (HLB: less than 11, preferably less than 8, more preferably less than 5) and amino-modified silicones are preferable, with the amino-modified silicones being more preferable.

Examples of the dimethylpolysiloxanes include the compounds represented by the general formula (b1):

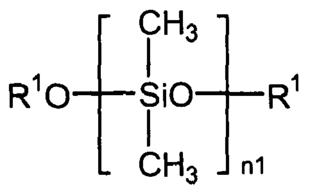

(b1)

wherein $R^1$ represents a $Si(CH_3)_3$ or a hydrogen atom, and $n^1$ represents a number of 3 to 20000.

Dimethylpolysiloxanes include silicone oils (number average polymerization degree: less than 1000) and silicone rubbers (number average polymerization degree: 1000 or more). Commercially available examples of the silicone oils include: the SH200 series (SH200 C Fluid 1CS, 2CS, 5CS, 10CS, 20CS, 30CS, 50CS, 100Cs, 200CS, 350CS, 500CS, 1,000CS, 5,000CS; SH200 Fluid 1.5CS, 3,000CS, 10,000CS, 12,500CS, 30,000CS and others) (products of Dow Corning Toray Co., Ltd.); TSF-451 series (products of GE Toshiba Silicones Co., Ltd.); and KF-96 series (products of Shin-Etsu Chemical Co., Ltd.). Additionally, emulsified products of these silicone oils may also be used.

Commercially available examples of the silicone rubbers include: SH200 series (SH200 Fluid 60,000CS, 100,000CS, 1,000,000CS and others; products of Dow Corning Toray Co., Ltd.); TSF451-100MA (product of GE Toshiba Silicones Co., Ltd.); BY11-026 (product of Dow Corning Toray Co., Ltd.; a solution of a highly polymerized silicone diluted with a low-viscosity silicone); KF9008 (product of Shin-Etsu Chemical Co., Ltd.; a solution of a highly polymerized silicone diluted with a cyclic silicone); BY-22-050A (product of Dow Corning Toray Co., Ltd.; a cationic emulsion of a highly polymerized silicone); BY22-060 (product of Dow Corning Toray Co., Ltd.; a cationic emulsion of a solution of a highly polymerized silicone diluted with a low-viscosity silicone); BY22-020 (product of Dow Corning Toray Co., Ltd.; a cationic emulsion of a solution of a highly polymerized silicone diluted with a liquid paraffin); and KM904 (product of Shin-Etsu Chemical Co., Ltd.; a cationic emulsion of a solution of a highly polymerized silicone diluted with a low-viscosity silicone).

Polyether-modified silicone is a generic designation for polyoxyethylene/methylpolysiloxane copolymer, poly(oxyethylene/oxypropylene)methylpolysiloxane copolymer and others; examples of the polyether-modified silicone include the polymers represented by the general formula (b2) or (b3) :

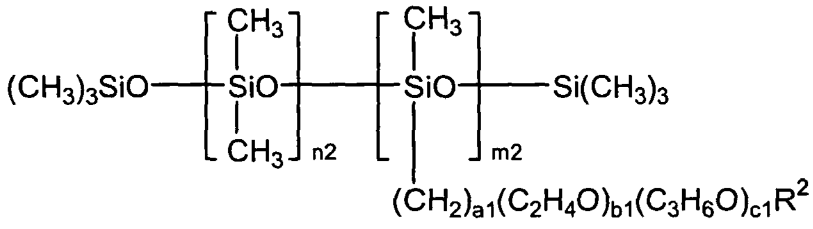

(b2)

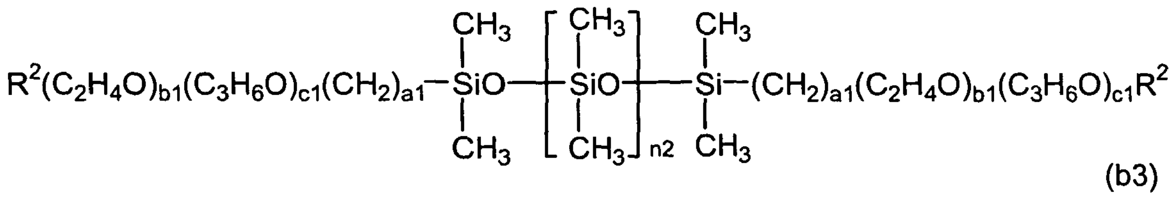

(b3)

wherein $R^2$ represents a hydrogen atom or an alkyl group having 1 to 12 carbon atoms, $n^2$ represents a number of 1 to 2000, $m^2$ represents a number of 1 to 1000, $a^1$ represents a number of 0 to 10, $b^1$ represents a number of 0 to 50, $c^1$ represents a number of 0 to 50, and $b^1 + c^1 \geq 1$.

Commercially available examples of the polyether-modified silicones with HLB less than 11 include: SH3775M and SS-2805 (products of Dow Corning Toray Co., Ltd.); KF-6015, KF-6016, KF-6017 and KF-6029 (products of Shin-Etsu Chemical Co., Ltd.).

An amino-modified silicone may be a silicone which includes an amino group or an ammonium group. The amino-modified silicone may be either an amino-modified silicone oil in which all or part of the terminal hydroxy groups are blocked with methyl groups or the like, or amodimethicone in which no terminals are blocked. Examples of the amino-modified silicone include the silicones represented by the following general formula (b4) or (b5). Additionally, poly(N-acylalkyleneimine)-modified silicones may also be suitably used.

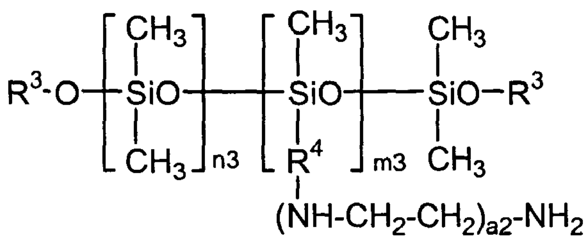

(b4)

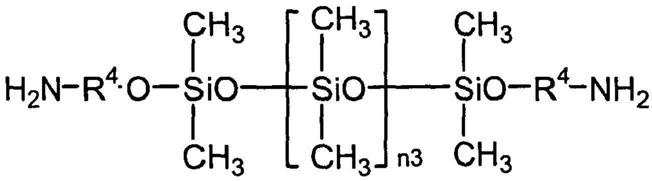

(b5)

wherein $R^3$ represents $Si(CH_3)_3$ or a hydrogen atom, $R^4$ represents an alkylene group having 2 to 8 carbon atoms, $n^3$ represents a number of 1 to 20000, $m^3$ represents a number of 1 to 2000, and $a^2$ represents a number of 0 to 3; and the nitrogen content is preferably 0.02 to 4% by weight and more preferably 0.1 to 1% by weight.

Commercially available examples of the amino-modified silicones include: amino-modified silicone oils such

as SF8451C (product of Dow Corning Toray Co., Ltd.; viscosity: 600 $mm^2/s$; nitrogen content: 0.8% by weight), SF8452C (product of Dow Corning Toray Co., Ltd.; viscosity: 700 $mm^2/s$; nitrogen content: 0.2% by weight), SF8457C (product of Dow Corning Toray Co., Ltd.; viscosity: 1200 $mm^2/s$; nitrogen content: 0.8% by weight), KF8003 (product of Shin-Etsu Chemical Co., Ltd.; viscosity: 1850 $mm^2/s$; nitrogen content: 0.7% by weight), KF8005 (product of Shin-Etsu Chemical Co., Ltd.; viscosity: 1200 $mm^2/s$; nitrogen content: 0.1% by weight), KF867 (product of Shin-Etsu Chemical Co., Ltd.; viscosity: 1300 $mm^2/s$; nitrogen content: 0.8% by weight) and KF8012 (product of Shin-Etsu Chemical Co., Ltd.; viscosity: 90 $mm^2/s$; nitrogen content: 0.6% by weight); and amodimethicone emulsions such as SM8704C (product of Dow Corning Toray Co., Ltd.; nitrogen content: 0.8% by weight), SM8904C (product of Dow Corning Toray Co., Ltd.; nitrogen content: 0.3% by weight) and BY22-079 (product of Dow Corning Toray Co., Ltd.; nitrogen content: 0.6% by weight). Additionally, preferably usable examples also include CF1046 (product of Dow Corning Toray Co., Ltd.; nitrogen content: 0.14% by weight) which is a mixture (weight ratio: 10:3.7:2.9) composed of dimethylpolysiloxane (number average polymerization degree: 550), dimethylpolysiloxane (number average polymerization degree: 2700) and an amino-modified silicone.

A poly(N-acylalkyleneimine)-modified silicone is a silicone formed by bonding the segment of poly(N-acylalkyleneimine) composed of a repeating unit represented by the following general formula (b6) to an organopolysiloxane segment in such a way that the bonding occurs to at least one of the silicon atoms in the organopolysiloxane segment through the hetero atom-containing alkylene group:

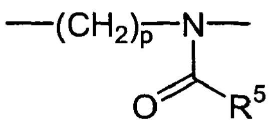

(b6)

wherein $R^5$ represents a hydrogen atom, an alkyl group having 1 to 22 carbon atoms, a cycloalkyl group, an aralkyl group or an aryl group, and p represents a number of 2 or 3. The alkyl group represented by $R^5$ is an alkyl group preferably having 1 to 20 carbon atoms, more preferably 1 to 5 carbon atoms, and even more preferably 1 or 2 carbon atoms. Examples of the cycloalkyl group include the cycloalkyl groups having 3 to 6 carbon atoms; examples of the aralkyl group include a phenylalkyl group and naphthylalkyl group; and examples of the aryl group include a phenyl group, a naphthyl group and an alkylsubstituted phenyl group. $R^5$ is preferably a methyl group or an ethyl group.

In the poly(N-acylalkyleneimine)-modified silicone, the weight ratio of the organopolysiloxane segment to the poly(N-acylalkyleneimine) segment is 98/2 to 40/60, and is preferably 94/6 to 60/40; and the weight average molecular weight of the poly(N-acylalkyleneimine)-modified silicone is 50,000 to 500,000, and is preferably 100,000 to 300,000.

Examples of the hetero atom-containing alkylene group intermediating in the bond between the organopolysiloxane segment and the poly(N-acylalkyleneimine) include alkylene groups each having 2 to 20 carbon atoms wherein each alkylene group contains one to three nitrogen, oxygen and/or sulfur atoms. Examples of such a group include those represented by the following formulas:

$-(CH_2)_3-NH-$ $\quad$ $-(CH_2)_3-NH-(CH_2)_2-NH-$ $\quad$ $-(CH_2)_3-S-$

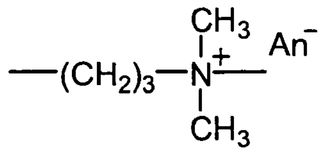

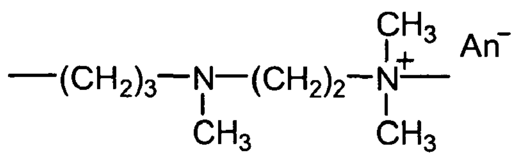

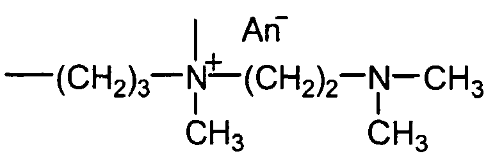

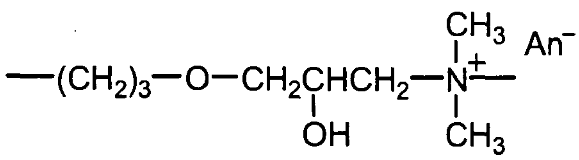

wherein $An^-$ represents an anion. Further, nitrogen atom-containing alkylene groups having 2 to 5 carbon atoms are preferable.

Poly(N-acylalkyleneimine)-modified silicone can be produced according to a known method such as the method disclosed in JP-A-7-133352, by reacting an organopolysiloxane represented by the following general formula (b6-a) with a terminal reactive poly(N-acylalkyleneimine obtained by ring-opening polymerization of a cyclic iminoether represented by the following general formula (b6'):

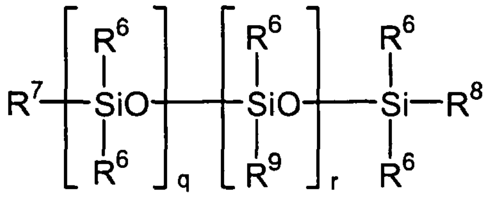

(b6-a)

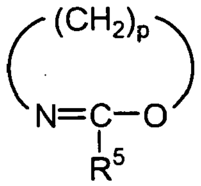

(b6')

wherein:

in formula (b6-a), $R^6$s are the same or different and each independently represent a saturated alkyl group having 1 to 22 carbon atoms or a phenyl group, $R^7$ and $R^8$ each represent the same group as $R^6$, or a group represented by any one of the following formulas, $R^9$ represents a group represented by any one of the following formulas, q represents an integer of 100 to 4000, and r represents an integer of 1 to 300:

$-(CH_2)_3-NH_2$ $-(CH_2)_3-NH-(CH_2)_2-NH_2$

$-(CH_2)_3-SH$

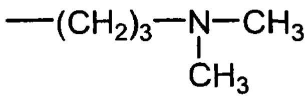

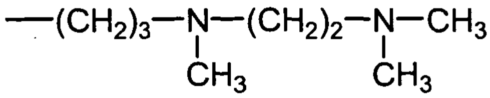

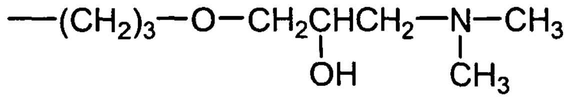

and in formula (b6'), $R^5$ and p are the same as described above.

It is to be noted that the ring opening polymerization of the cyclic iminoether (b6') can be carried out, for example, according to the method described in Liebigs Ann. Chem., pp. 996 to 1009 (1974). As the polymerization initiator, there may be used compounds having strong electrophilic reactivity such as methyl, ethyl, 3-propenyl or benzyl esters of strong acids such as benzenesulfonic acid, p-toluenesulfonic acid, trifluoromethane sulfonic acid, trifluoroacetic acid, and sulfuric acid. Specifically, alkyl toluenesulfonates, dialkyl sulfate, alkyl trifluoromethanesulfonates and the like may be preferably used. When 2-substituted-2-oxazoline is used as the cyclic iminoether (b6'), poly(N-acylethyleneimine) (corresponding to p = 2 in formula (b6)) is obtained. Alternatively, when 2-substituted-dihydro-2-oxazine is used, poly (N-acylpropyleneimirie) (corresponding to p = 3 in formula (b6)) is obtained. The molecular weight of the molecular chain of the above-described poly(N-acylalkyleneimine) is preferably 150 to 50,000 and more preferably 500 to 10,000.

As the method for linking the above-described poly(N-acylalkyleneimine) chain and a silicone chain, the following

various reaction methods may be employed: an ester formation reaction based on the condensation between a carboxyl group and a hydroxy group; an amide formation reaction based on the condensation of a carboxyl group and an amino group; a secondary, tertiary or quaternary ammonium formation reaction between a halogenated alkyl group and a primary, secondary or tertiary amino group; an addition reaction of a Si-H group to a vinyl group; and a β-hydroxyamine formation reaction involving an epoxy group and an amino group. Among them, as disclosed in JP-A-2-276824, JP-A-4-85334, JP-A-4-85335, JP-A-4-96933, simple and effective is a method in which a terminal reactive poly(N-acylalkyleneimine) obtained by a cationic ring-opening polymerization of a cyclic iminoether is reacted with an organopolysiloxane represented by formula (b6-a), namely, a modified organopolysiloxane having the above-described substituents in the side chains thereof.

The reaction between the amino group-containing organopolysiloxane and the reactive terminal of poly(N-acylalkyleneimine) obtained by a cationic polymerization of a cyclic iminoether can be carried out, for example, in the following manner. An initiator is dissolved in a polar solvent such as a single solvent preferably such as acetonitrile, valeronitrile, dimethylformamide, dimethylacetoamide, chloroform, methylene chloride, ethylene chloride and ethyl acetate or methyl acetate, or, according to need, in a mixed solvent composed of any one of these solvents and other solvents. The solution thus obtained is increased in temperature up to 40 to 150°C, preferably, 60 to 100°C. A cyclic iminoether represented by the above general formula (b6') is placed at a time in the solution, or added dropwise to the solution when the reaction is vigorous, and thus the polymerization is carried out. The progress of the polymerization can be traced by quantitatively determining the cyclic iminoether remaining as the monomer with an analytical instrument such as a gas chromatograph. Even when the cyclic iminoether is consumed and the reaction is completed, the active species at the growing terminal still maintains the reactivity. Successively, without isolating the polymer, this polymer solution and the organopolysiloxane containing amino groups in the molecule thereof are mixed together, and the mixture is allowed to react under the condition of 5 to 100°C, preferably, 20 to 60°C. The mixing ratio can be appropriately selected according to the desired purposes; however, the reaction is preferably carried out with 0.1 to 1.3 molar equivalent of poly(N-acylalkyleneimine) in relation to 1 mole of the amino groups of the organopolysiloxane. The reactions as described above enable to obtain a block copolymer or graft polymer in which poly(N-acylalkyleneimine)segments are bonded to polydimethylsiloxane.

Examples of the poly(N-acylalkyleneimine)-modified silicone include poly(N-formylethyleneimine)organosiloxane, poly(N-acetylethyleneimine)organosiloxane, and poly(N-propionylethyleneimine)organosiloxane.

The hydrophobic silicones as component (B) may be used each alone or in combination of two or more thereof. Among the hydrophobic silicones, amino-modified silicones are preferable, and poly(N-acylalkyleneimine)-modified silicones are more preferable.

The content of the component (B) is preferably 0.01 to 20% by weight, more preferably 0.1 to 10% by weight and even more preferably 0.5 to 5% by weight of the hair treatment agent used in the present invention, from the viewpoints of an advantageous effect that, at the time of aligning the hair streaks, hair is made smooth by decreasing the hair surface friction resistance of the wetted hair to suppress the hair entanglement and to thereby facilitate bundling, and another advantageous effect that a soft set coating is formed on the surface of the dried hair after finishing, and consequently the occurrence of loose hairs and hair bulging with time is suppressed to improve the retention of the hairstyle.

The hair treatment agent used in the present invention further includes a lower alcohol, as component (C). The lower alcohol as component (C) provides advantageous effects that the lower alcohol reduces the surface tension of the hair treatment agent to enable quick wetting of the hair, and after bundling the hair, enables the hair to be dried naturally and quickly even in the inside of the hair, and additionally, dissolves component (B) and the below-described component (F).

The lower alcohol is a straight chain or branched chain alcohol having 2 to 6 carbon atoms; specific examples of such an alcohol include ethanol, 1-propanol, 2-propanol and 1-butanol. Among them, ethanol and 2-propanol are preferable, and ethanol is more preferable.

The content of the lower alcohol is set to be at 0.5% by weight or more, and is preferably 1 to 50% by weight, more preferably 4 to 40% by weight and even more preferably 8 to 30% by weight of the hair treatment agent, from the viewpoint that the hair can be quickly wetted, and after bundling, the hair is dried naturally and quickly even in the inside of the hair.

The hair treatment agent used in the present invention further includes a surfactant as component (D). The surfactant as component (D) provides advantageous effects of the solubilization or emulsification of the below-described component (F), the stabilization of the system, and the improvement of the feel to the touch. As the surfactant, any of the following may be used: cationic surfactants, nonionic surfactants, amphoteric surfactants and anionic surfactants.

Examples of the cationic surfactant include quaternary ammonium salts represented by the following general formula (d1):

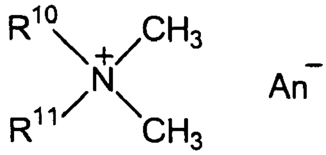

(d1)

wherein $R^{10}$ and $R^{11}$ each independently represent a hydrogen atom, an alkyl group having 1 to 28 carbon atoms or a benzyl group with the proviso that excluded are the case where $R^{10}$ and $R^{11}$ are each simultaneously a hydrogen atom or a benzyl group and the case where $R^{10}$ and $R^{11}$ are each simultaneously a lower alkyl group having 1 to 3 carbon atoms; and $An^-$ represents an anion.

As for $R^{10}$ and $R^{11}$, one of $R^{10}$ and $R^{11}$ is preferably a alkyl group having 16 to 24 carbon atoms, more preferably an alkyl group having 22 carbon atoms and even more preferably a straight chain alkyl group, and the other of $R^{10}$ and $R^{11}$ is preferably a lower alkyl group having 1 to 3 carbon atoms and more preferably a methyl group. As the anion $An^-$, preferable are halide ions such as a chloride ion and a bromide ion, and organic anions such as an ethyl sulfate ion and a methyl carbonate ion; halide ions are preferable and a chloride ion is more preferable.

As the cationic surfactant, a mono-long-chain-alkyl quaternary ammonium salt is preferable; specific examples of such a salt include: cetyltrimethylammonium chloride, stearyltrimethylammonium chloride, arachyltrimethylammonium chloride and behenyltrimethylammonium chloride; and stearyltrimethylammonium chloride and behenyltrimethylammonium chloride are more preferable.

Examples of the nonionic surfactant include polyoxyalkylene alkyl ethers, polyoxyalkylene alkenyl ethers, higher fatty acid sucrose esters, polyglycerin fatty acid esters, higher fatty acid mono- or diethanolamides, polyoxyethylene hydrogenated castor oil, polyoxyethylene sorbitan fatty acid esters, polyoxyethylene sorbitol fatty acid esters, alkylsaccharide surfactants, alkylamine oxides, and alkylamidoamine oxides. Among them, polyoxyalkylene alkyl ethers and polyoxyethylene hydrogenated castor oil are preferable, with polyoxyethylene alkyl ethers being more preferable.

Examples of the amphoteric surfactant include imidazoline, carbobetaine, amidobetaine, sulfobetaine, hydroxysulfobetaine, and amidosulfobetaine surfactants.

Examples of the anionic surfactant include alkylbenzenesulfonates, alkyl or alkenyl ether sulfates, alkyl or alkenyl sulfates, olefinsulfonates, alkanesulfonates, salts of saturated or unsaturated fatty acids, alkyl or alkenyl ether carbonates, salts of $\alpha$-sulfone fatty acids, N-acylamino acid surfactants, phosphoric acid monoester or diester surfactants and sulfosuccinic acid esters. Examples of the counter ions of the anionic residues of the above-described surfactants include: alkali metal ions such as a sodium ion and a potassium ion; alkali earth metal ions such as a calcium ion and a magnesium ion; ammonium ion; and alkanolamines having 1 to 3 alkanol groups each having 2 or 3 carbon atoms (for example, monoethanolamine, diethanolamine, triethanolamine and triisopropanolamine). Additionally, examples of the counter ions of the cationic residues include: halide ions such as a chloride ion, a bromide ion and an iodide ion; a methosulfate ion; and a saccharinate ion.

From the viewpoint of feel to the touch, preferable among them are cationic surfactants. The surfactants may be used each alone or in combination of two or more thereof. The content of the surfactant(s) is preferably 0.01 to 10% by weight, more preferably 0.05 to 3% by weight and even more preferably 0.1 to 2% by weight of the hair treatment agent used in the present invention, from the viewpoints of the feel to the touch, the solubilization or the emulsification of the solvent and the stability of the system.

The hair treatment agent used in the present invention preferably further includes an organic carboxylic acid as component (E), from the viewpoint that the humidity resistance of the hair is improved, the occurrence of loose hairs/flyaway hairs from the gathered hair style after drying is suppressed, and thus the retention of the hairstyle is improved. As the organic carboxylic acid as component (E), preferable are hydroxycarboxylic acids, dicarboxylic acids, tricarboxylic acids and acidic amino acids. Specific examples of such carboxylic acids include: hydroxycarboxylic acids such as glycolic acid, lactic acid, malic acid, tartaric acid and citric acid; dicarboxylic acids such as malonic acid, succinic acid, glutaric acid, adipic acid, maleic acid, fumaric acid, phthalic acid, oxalic acid, malic acid and tartaric acid; as tricarboxylic acids, citric acid; and acidic amino acids such as glutamic acid and aspartic acid.
Preferable among them are malic acid, tartaric acid, malonic acid, succinic acid, maleic acid and lactic acid, and more preferable are malic acid and lactic acid. Additionally, examples of the salts of these organic carboxylic acids include the salts with alkali metals, alkali earth metals, ammonia and organic amine compounds.

These compounds as component (E) may be used in combination of two or more thereof, and the content of the compound(s) as component (E) is preferably 0.1 to 30% by weight, more preferably 0.5 to 20% by weight and even more preferably 0.5 to 10% by weight of the hair treatment agent.

The hair treatment agent used in the present invention preferably further includes an organic solvent, as a component (F), selected from the following (f1) to (f5), from the viewpoint that the humidity resistance of the hair is

improved, the occurrence of loose hairs/flyaway hairs from the gathered hair style after drying is suppressed, and thus the retention of the hairstyle is improved.

(f1) An aromatic alcohol represented by the general formula (f1):

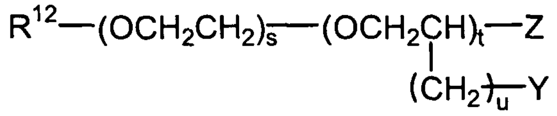

(f1)

wherein $R^{12}$ represents a group $R^{13}$-Ph-$R^{14}$- ($R^{13}$: a hydrogen atom, a methyl group or a methoxy group; $R^{14}$: a bond or a divalent saturated or unsaturated hydrocarbon group having 1 to 3 carbon atoms; Ph: a paraphenylene group); Y and Z each represent a hydrogen atom or a hydroxy group; and s, t and u each represent an integer of 0 to 5; with the proviso that when s = t = 0, Z is a hydroxy group and $R^{12}$ is not a $R^{13}$-Ph- group.
(f2) N-Alkylpyrrolidone or N-alkenylpyrrolidone in which an alkyl group or an alkenyl group having 1 to 18 carbon atoms is bonded to the nitrogen atom, respectively.
(f3) An alkylene carbonate having 2 to 4 carbon atoms.
(f4) A polypropylene glycol having a number average molecular weight of 100 to 1000.
(f5) A lactone or a cyclic ketone represented by the general formula (f5-a), (f5-b) or (f5-c):

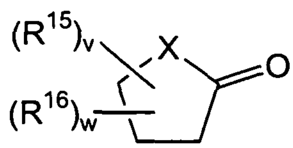

(f5-a)

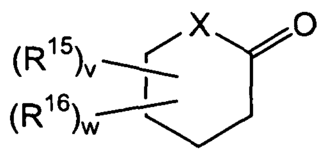

(f5-b)

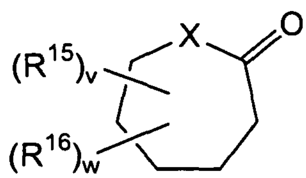

(f5-c)

wherein X represents a methylene group or an oxygen atom, $R^{15}$ and $R^{16}$ represent different substituents, and v and w each represent 0 or 1.

Among the organic solvents as component (F), examples of (f1) include benzyl alcohol, cinnamyl alcohol, phenethyl alcohol, p-anisyl alcohol, p-methylbenzyl alcohol, phenoxyethanol and 2-benzyloxyethanol. Examples of (f2) include N-methylpyrrolidone, N-octylpyrrolidone and N-laurylpyrrolidone. Examples of (f3) include ethylene carbonate and propylene carbonate. As the polypropylene glycol of (f4) having a number average molecular weight of 100 to 1000, preferable is a polypropylene glycol having a number average molecular weight of 100 to 500 and additionally having a polymerization degree of 2 to 5. In the (f5) compounds, preferable examples of $R^{15}$ and $R^{16}$ in the general formulas (f5-a) to (f5-c) include straight chain, branched chain or cyclic alkyl groups, a hydroxy group, a sulfonic acid group, a phosphoric acid group, a carboxy group, a phenyl group, a sulfoalkyl group, an alkyl phosphate group and a carboxyalkyl group; preferable among them is a straight chain or branched chain alkyl group having 1 to 6 carbon atoms such as a methyl group, an ethyl group, a propyl group, an isopropyl group or a butyl group, substituted at the $\gamma$ position in the case of a $\gamma$-lactone and at the $\delta$ position in the case of a $\delta$-lactone (namely, in the methylene adjacent to the hetero oxygen atom). Additionally, when the water-solubilities of the compounds (f5-a) to (f5-c) are desired to be increased, preferable are the compounds each of which has, as $R^{15}$ or $R^{16}$, an acidic group such as a sulfonic acid group, a phosphoric acid group or a carboxy group, or an alkyl group substituted with any one of these acidic groups. Among the (f5) compounds, examples of the lactone include $\gamma$-butyrolactone, $\gamma$-caprolactone, $\gamma$-valerolactone, $\delta$-valerolactone, $\delta$-caprolactone and $\delta$-heptanolactone; from the viewpoint of the stability of the lactones, $\gamma$-lactones are preferable, and further, $\gamma$-butyrolactone and $\gamma$-caprolactone are preferable. Among the (f5) compounds, examples of the cyclic ketone include cyclopentanone, cyclohexanone, cycloheptanone and 4-methylcycloheptanone.
Examples of the preferable compounds as component (F) include benzyl alcohol, benzyloxyethanol, propylene carbonate and propylene glycol (number average molecular weight: 300 to 500, preferably, 400).
Additionally, component (F) is preferably liquid at 25°C, is preferred that ClogP be -2 to 3, and ClogP is preferably -1 to 2 from the viewpoint of promoting penetration into the hair. It is to be noted that ClogP is a calculated value of the

octanol-water-partition coefficient (logP) defined by the following formula as an index indicating a measure of the partition of a substance between an octanol phase and a water phase, and some examples of such calculations are described in Chemical Reviews, Vol. 71, No. 6 (1971):

$$\log P = \log([\text{substance}]_{\text{Octanol}} / [\text{substance}]_{\text{Water}})$$

wherein $[\text{substance}]_{\text{Octanol}}$ represents the molar concentration of the substance in the 1-octanol phase and $[\text{substance}]_{\text{Water}}$ represents the molar concentration of the substance in the water phase.

Specific examples of the ClogP values for the main compounds as the component (F) are as follows: benzyl alcohol (1.1), 2-benzyloxyethanol (1.2), 2-phenylethanol (1.2), 1-phenoxy-2-propanol (1.1), polypropylene glycol 400 (0.9), propylene carbonate (-0.41), and γ-butyrolactone (-0.64).

The compounds as the component (F) may be used in combination of two or more thereof; and the content of the compound(s) as component (F) is preferably 0.1 to 40% by weight, more preferably 0.5 to 10% by weight, and even more preferably 1 to 5% by weight of the hair treatment agent.

Additionally, the hair treatment agent used in the present invention may include a polyhydric alcohol. The polyhydric alcohol contributes to the solubilization and the stable dispersion of component (F), and works synergically with component (F) to improve the hair-modifying effects and to impart gloss to the hair. Examples of the polyhydric alcohol include ethylene glycol, glycerin, sorbitol, propylene glycol and 1,3-butylene glycol; preferable among them is glycerin. The polyhydric alcohols may be used each alone or in combination of two or more thereof, and the content of the polyhydric alcohol(s) is preferably 0.1 to 10% by weight and more preferably 0.5 to 5% by weight of the hair treatment agent used in the present invention.

The hair treatment agent used in the present invention may further include a set polymer from the viewpoint of easy alignment of hair steaks at the time of forming gathered hair, retention improvement of the hairstyle of the gathered hair, viscosity adjustment and the stability. Examples of such a polymer include: polyvinylpyrrolidone polymer compounds such as polyvinylpyrrolidone, vinylpyrrolidone/vinyl acetate copolymer, vinylpyrrolidone/vinyl acetate/vinyl propionate ternary copolymer, vinylpyrrolidone/alkylaminoacrylate (quaternized chloride) copolymer, vinylpyrrolidone/acrylate/(meth)acrylic acid copolymer and vinylpyrrolidone/alkylaminoacrylate/vinylcaprolactam copolymer; acidic vinyl ether polymer compounds such as methyl vinyl ether/maleic anhydride alkyl half ester copolymer; acidic polyvinyl acetate polymer compounds such as vinyl acetate/crotonic acid copolymer, vinyl acetate/crotonic acid/vinyl neodecanoate copolymer and vinyl acetate/crotonic acid/vinyl propionate copolymer; acidic acrylic polymer compounds such as (meth) acrylic acid/(meth)acrylate copolymer and acrylic acid/alkyl acrylate/alkylacrylamide copolymer; amphoteric acrylic polymer compounds such as N-methacryloylethyl-N,N-dimethylammonium-α-N-methylcarboxybetaine/butyl methacrylate copolymer and hydroxypropyl acrylate/butylaminoethyl methacrylate/octyl acrylate amide copolymer; basic acrylic polymer compounds such as acrylamide acrylate quaternary copolymer; cellulose derivatives such as cationic cellulose derivative; and chitin-chitosan derivatives such as hydroxypropyl chitosan, carboxymethyl chitin and carboxymethyl chitosan.

These set polymers may be used each alone or in combination of two or more thereof, and the content of the set polymer(s) is preferably 0.01 to 10% by weight, more preferably 0.04 to 5% by weight, even more preferably 0.08 to 2% by weight and even more preferably 0.08 to 1% by weight of the hair treatment agent used in the present invention.

The hair treatment agent used in the present invention may further include components employed for ordinary hair cosmetic compositions, in addition to the above described components, according to the intended applications. Examples of such components include antidandruffs; vitamin preparations; bactericides; antiinflammatories; chelating agents; humectants such as sorbitol and panthenol; coloring agents such as dyes and pigments; viscosity modifiers such as hydroxyethyl cellulose, methyl cellulose, polyethylene glycol and clay mineral; pH adjusters such as organic acids, sodium hydroxide and potassium hydroxide; plant extracts; pearling agents; perfumes; colorants; ultraviolet absorbers; antioxidants; olea such as hydrocarbons, glycerides, higher alcohols, ester oils and higher fatty acids; and additionally, those components described in ENCYCLOPEDIA OF SHAMPOO INGREDIENTS (MICELLE PRESS).

The hair treatment agent used in the present invention has a pH, at 25°C under 20-fold by weight dilution with water, falling within a range preferably from 2.5 to 4.5, more preferably from 2.5 to 4 and even more preferably from 3 to 4, from the viewpoint that the humidity resistance of the hair is improved, the occurrence of loose hairs/flyaway hairs from the gathered hair style after drying is suppressed, and thus the retention of the hairstyle is improved.

The following examples further describe and demonstrate embodiments of the present invention. The examples are given solely for the purpose of illustration and are not to be construed as limitations of the present invention.

Examples

Examples 1 to 4 and Comparative Examples 1 to 7
As a first hair treatment agent, each of the hair treatment agents shown in Table 1 was placed in a pump mist container (pump sprayer YSR; manufactured by Yoshino Kogyo Co., Ltd.; 0.16 g/push) to obtain a pump mist. It is to be noted that Comparative Specimen 1 is water. Each of the hair treatment agents was subjected to the evaluation of "stability," "easiness in spreading on the hair," "easiness in fixing hair roots," and "easiness in combing," on the basis of the below-described standards. The results thus obtained are shown in Table 1.

(Evaluation Standards for "Stability")

The directly-after stability was evaluated by observing the appearance of each hair treatment agent after having been allowed to stand still at room temperature for 6 hours. The low-temperature stability was evaluated by observing the appearance of each hair treatment agent after having been allowed to stand still at -5°C for one week.
A: No separation/precipitation is observed.
B: Almost no separation/precipitation is observed.
C: Slight separation/precipitation is observed.
D: Separation/precipitation is observed.

(Evaluation Standards for "Easiness in Spreading Treatment Agent on Hair," "Easiness in Fixing Hair Roots," and "Easiness in Combing")

Evaluation was carried out by a panel of five experts as follows: each expert gave one of the marks 1 to 5, and the total sum of the marks given by the five experts was obtained; the cases where the total sum was 20 to 25, 15 to 19, 10 to 14, and 5 to 9 were marked with A, B, C and D, respectively, in Table 1.
TABLE 1

| Components | | Ex. product | | Comparative product | | | | |
|---|---|---|---|---|---|---|---|---|
| | | 1 | 2 | 1 | 2 | 3 | 4 | 5 |
| Oxazoline-modified organopolysiloxane (*1) | | 0.60 | 0.60 | - | 0.60 | 0.60 | - | - |
| Amino-modified silicone emulsion (*2) | | 0.40 | 0.40 | - | 0.40 | 0.40 | - | - |
| Polyether-modified silicone (*3) | | 0.50 | 0.50 | - | 0.50 | 0.50 | - | - |
| Polyether-modified silicone (*4) | | - | - | | - | - | - | 1.20 |
| Liquid malic acid (50% by weight) | | 2.50 | 2.50 | - | 2.50 | 2.50 | 2.50 | 2.50 |
| Lactic acid (90% by weight) | | 1.39 | 1.39 | - | 1.39 | 1.39 | 1.39 | 1.39 |
| Benzyl alcohol | | 1.25 | - | - | 1.25 | 1.25 | 1.25 | 1.25 |
| Benzyloxyethanol | | - | 1.25 | - | - | - | - | - |
| Ethanol solution of polyquaternium-11(*5) | | 0.20 | 0.20 | - | 0.20 | 0.20 | 0.20 | 0.20 |
| Aqueous solution of stearyltrimethylammonium chloride (28% by weight) | | 0.90 | 0.90 | - | - | 0.90 | 0.90 | 0.90 |
| PEG-32 | | 0.20 | 0.20 | - | 0.20 | 0.20 | 0.20 | 0.20 |
| Ethanol (95% by volume) | | 19.50 | 19.50 | - | 19.50 | - | 19.50 | 19.50 |
| Concentrated glycerin | | 2.00 | 2.00 | - | 2.00 | 2.00 | 2.00 | 2.00 |
| Citron extract | | 0.05 | 0.05 | - | 0.05 | 0.05 | 0.05 | 0.05 |
| Oxybenzone-9 | | 0.05 | 0.05 | - | 0.05 | 0.05 | 0.05 | 0.05 |
| Disodium edetate | | 0.05 | 0.05 | - | 0.05 | 0.05 | 0.05 | 0.05 |
| Perfume | | 0.05 | 0.05 | - | 0.05 | 0.05 | 0.05 | 0.05 |
| Purified water | | Bal. | Bal. | 100.00 | Bal. | Bal. | Bal. | Bal. |
| Sodium hydroxide (pH adjuster) | | q.s. | q.s. | - | q.s. | q.s. | q.s. | q.s. |
| pH (diluted 20-fold by weight with water, 25°C) | | 3.7 | 3.7 | - | 3.7 | 3.7 | 3.7 | 3.7 |
| Viscosity (mPa·s) | | 4.2 | 4.0 | 1 | 3.7 | 3.9 | 2.4 | 2.8 |
| Evaluation | Directly-after stability | A | A | A | A | D | A | A |
| | Low-temperature stability | A | A | D | C | D | A | A |
| | Easiness in spreading treatment agent | A | A | D | B | C | B | B |
| | Easiness in fixing hair roots | A | A | C | B | C | B | B |
| | Easiness in combing | A | A | D | C | C | C | C |

*1: Elastomer-OS-88, Kao Corp.

*2: Amino-modified silicone SM8704C, Dow Corning Toray Co., Ltd., act. 40% by weight (hydrophobic)

*3: KF-6029, Shin-Etsu Chemical Co. Ltd., HLB 10, act. 90% by weight (hydrophobic)

*4: KF-6011, Shin-Etsu Chemical Co. Ltd., HLB 14.5 (hydrophilic)

*5: Gafquat 734, ISP Japan Co., Ltd., act. 50% by weight

As a second hair treatment agent, each of the hair wax (treatment agent W) shown in Table 2 and the hair spray (treatment agent S) shown in Table 3 was prepared.

TABLE 2

| Components | Treatment agent W (% by weight) |
|---|---|
| Cetostearyl alcohol | 4.0 |
| Praffin (m.p. 53°C) | 6.5 |
| Praffin (m.p. 69°C) | 10.0 |
| Microcrystalline wax (*1) | 6.5 |
| Vaseline | 10.0 |
| Candelilla wax/rice bran wax/paraffin mixture | 2.0 |
| Liquid paraffin | 3.0 |
| Polyoxyethylene cetyl ether (20E.O.) | 5.0 |
| Self-emulsification type glycerin monostearate | 3.0 |
| Sodium polyoxyethylene lauryl ether phosphate | 0.5 |
| Propyl paraoxybenzoate | 0.1 |
| Octyldodecyl myristate | 3.0 |
| Carboxyvinyl polymer | 0.1 |
| Polyethylene glycol 1540 | 5.0 |
| Sodium N-stearoyl-N-methyltaurine | 1.0 |
| Methyl paraoxybenzoate | 0.2 |
| Butylene glycol | 5.0 |
| Sodium dehydroacetate | 0.0 |
| Disodium edetate | 0.1 |
| Polyvinylpyrrolidone | 0.80 |
| Sodium benzoate | 0.20 |
| Perfume | 0.10 |
| Purified water | Balance |
| Sodium hydroxide (pH adjuster) | q.s. |
| pH | 7.00 |
| *1:Multiwax W-445(Crompton Corp.) | |

TABLE 3

| Components | Treatment agent S (% by weight) |
|---|---|
| Set polymer(*1) | 2.50 |

(continued)

| Components | Treatment agent S (% by weight) |
|---|---|
| Lactic acid | 0.072 |
| Dimethylpolysiloxane | 0.30 |
| Perfume | 0.15 |
| Anhydrous ethanol | Balance |
| LPG (ejection agent) | 50.00 |
| Total | 100.0 |
| *1: RP77T, Kao Corp. | |

(Evaluation Methods for "Unlikeliness of Occurrence of Loose Hairs/Flyaway Hairs" and "Easiness in Drying Finished Gathered Hair")

Evaluation was carried out by a panel of five experts by using a long hair wig (product of Beaulax Co., Ltd.; No. 775S) as follows: each expert gave one of the marks 1 to 5, and the total sum of the marks given by the five experts was obtained; the cases where the total sum was 20 to 25, 15 to 19, 10 to 14, and 5 to 9 were marked with A, B, C and D, respectively, in Table 4.

Examples 1 and 2, and Comparative Examples 1 to 5

The hair around the top of the wig was lifted up, and each of the hair treatment agents described in Table 1 was applied with three pushes to the area between the hair roots and the portion to be bundled with a holding device, and then the lifted up hair bundle was allowed to hang down. This operation was repeated six times at around the same hair portion. In this way, the bath ratio was approximately such that agent:hair = 0.2:1. The hair streaks were aligned, and immediately thereafter the hair was bundled and secured with a holding device (a rubber band).

In the course of this process, the easiness in spreading the treatment agent on the hair, the easiness in fixing hair roots, the easiness in combing and the unlikeliness of occurrence of loose hairs/flyaway hairs (until securing, immediately after securing, and after 12 hours from securing) were evaluated.

Comparative Examples 6 and 7

The following two cases were evaluated: a case as Comparative Example 6 where the hair was applied with a hair treatment agent (Example product 1), combed, then dried with a dryer, and then set; and a case as Comparative Example 7 where the hair in a dried state was bundled and secured with a holding device.

Example 3

Evaluation was made in the same manner as in Example 1 on a case where by using the Example product 1 in Table 1, the hair treatment was carried out in the same manner as in Example 1, and then, the hair wax (treatment agent W) described in Table 2 was applied, as the second hair treatment agent, to the area between the hair roots and the portion bundled with a holding device.

Example 4

Evaluation was made in the same manner as in Example 1 on a case where by using the Example product 1 in Table 1, the hair treatment was carried out in the same manner as in Example 1, and then, the hair spray (treatment agent S) described in Table 3 was applied, as the second hair treatment agent, to the area between the hair roots and the portion bundled with a holding device.

TABLE 4

| | | Comparative Examples | | | | | | | Examples | | | |
|---|---|---|---|---|---|---|---|---|---|---|---|---|
| | | 1 | 2 | 3 | 4 | 5 | 6 | 7 | 1 | 2 | 3 | 4 |
| Treatment Methods | A: Dried hair was bundled and secured with a device<br>B: Hair was wetted with purified water, then combed, then bundled and secured with a device<br>C: Hair was applied with a hair treatment agent, combed, then bundled and secured with a device.<br>D: Hair was applied with a hair treatment agent, combed, dried with a dryer, then bundled and secured with a device<br>E: Hair treatment method C was applied, and then hair was set with a second hair treatment agent | B | C | C | C | C | D | A | C | C | E | E |
| Treatment agents | Hair treatment agent<br>CP: Comparative product<br>EP: Example product | CP 1 | CP 2 | CP 3 | CP 4 | CP 5 | EP 1 | - | EP 1 | EP 2 | EP 1 | EP 1 |
| | Second hair treatment agent | - | - | - | - | - | - | - | - | - | W | S |
| Evaluations | Unlikeliness of occurrence of loose hairs/flyaway hairs | | | | | | | | | | | |
| | Untill securing | C | B | C | C | C | D | D | A | A | A | A |
| | Immediately after securing | C | B | C | C | C | D | D | A | A | A | A |
| | After 12 hours from securing | D | C | C | C | C | D | D | B | B | A | A |
| | Easiness in drying finished gathered hair | D | B | D | B | B | A | - | A | A | A | A |

## Claims

1. A hair treatment method in which a hair treatment agent comprising (A) water, (B) a hydrophobic silicone, (C) 0.5 by mass or more of a lower alcohol and (D) a surfactant is applied to the hair, and immediately thereafter the hair is bundled and secured with a holding device.

2. The hair treatment method according to claim 1, wherein the hair treatment agent is applied to the hair in the area between the hair roots and the portion to be bundled with a holding device.

3. The hair treatment method according to claim 1 or 2, wherein the hair treatment agent is applied as a mist to the hair.

4. The hair treatment method according to any of claims 1 to 3, wherein the viscosity of the hair treatment agent is 100 mPa·s or less.

5. The hair treatment method according to any of claims 1 to 4, wherein after the hair is secured with a holding device, the secured hair is further treated with a second hair treatment agent.

6. The hair treatment method according to any one of claims 1 to 5, wherein (B) the hydrophobic silicone is selected from dimethylpolysiloxane, a polyether-modified silicone with HLB less than 11, an amino-modified silicone, a carboxy-modified silicone, methylphenylpolysiloxane, a fatty acid-modified silicone, an alcohol-modified silicone, an aliphatic alcohol-modified silicone, an epoxy-modified silicone, a fluorine-modified silicone, a cyclic silicone and an alkyl-modified silicone.

**7.** The hair treatment method according to any of claims 1 to 6, wherein (D) the surfactant is a cationic surfactant.

**8.** The hair treatment method according to any of claims 1 to 7, wherein the hair treatment agent further comprises (E) an organic carboxylic acid or a salt thereof, and the pH of the hair treatment agent is 2 to 7.

**9.** The hair treatment method according to any of claims 1 to 8, wherein the hair treatment agent further comprises (F) an organic solvent selected from the following (f1) to (f5) :

(f1) an aromatic alcohol;
(f2) an N-alkylpyrrolidone;
(f3) an alkylene carbonate;
(f4) propylene glycol or polypropylene glycol; and
(f5) a lactone or a cyclic ketone.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 7133352 A
- JP 2276824 A
- JP 4085334 A
- JP 4085335 A
- JP 4096933 A


### Non-patent literature cited in the description


- *Liebigs Ann. Chem.,* 1974, 996-1009
- *Chemical Reviews,* 1971, vol. 71 (6
- ENCYCLOPEDIA OF SHAMPOO INGREDIENTS. MICELLE PRESS